# EUROPEAN PATENT SPECIFICATION

(11) **EP 2 755 571 B1**
(45) Date of publication and mention of the grant of the patent: **01.01.2020**
(21) Application number: 12762154.8
(22) Date of filing: 10.09.2012
(51) Int. Cl.: A61B 17/072

(54) **SURGICAL INSTRUMENT AND BUTTRESS MATERIAL**
CHIRURGISCHES INSTRUMENT UND VERSTEIFUNGSMATERIAL
INSTRUMENT CHIRURGICAL ET MATÉRIAU DE CONTREFORT

(30) Priority: 15.09.2011 US 201113233664
(43) Date of publication of application: 23.07.2014
(73) Proprietor: Ethicon Endo-Surgery, Inc., Cincinnati, OH 45242 (US)
(72) Inventor: VASUDEVAN, Venkataramanan Mandakolathur, Cincinnati, Ohio 45249 (US); HUNT, John V., Cincinnati, Ohio 45241 (US); HUEIL, Geoffrey C., Mason, Ohio 45040 (US); NUR, Israel, Moshav Timmorim 79860 (IL); FANUELE, Greg J., Liberty Township, Ohio 45011 (US); KRAIMER, Joseph B., Mason, Ohio 45040 (US)
(74) Representative: Tunstall, Christopher Stephen
(86) International application number: PCT/US2012/054412
(87) International publication number: WO 2013/039825

(56) References cited:
- EP-A1- 1 994 890
- EP-A1- 2 090 237
- WO-A1-2009/143331
- DE-A1- 19 924 311
- US-A1- 2006 173 470
- US-A1- 2008 308 608

## Description

### BACKGROUND

US 2006/0173470 A1 describes an apparatus for equipping a surgical fastener with a buttress material. The apparatus is adapted to incorporate articles of buttress material and is configured for use with a linear stapler. The apparatus includes an alignment frame and an internally disposed pressure equalization member. The alignment frame can be configured in any way so as to retain articles of buttress material in releasable attachment within the aligning frame such that the articles will be automatically lined up with apposed jaw members of the linear stapler.

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through a cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasound, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient. Positioning of an end effector may be further facilitated through inclusion of one or more articulation joints or features, enabling the end effector to be selectively articulated or otherwise deflected relative to the longitudinal axis of the shaft.

Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Merely exemplary surgical staplers are disclosed in U.S. Pat. No. 4,805,823, entitled "Pocket Configuration for Internal Organ Staplers," issued February 21, 1989; U.S. Pat. No. 5,415,334, entitled "Surgical Stapler and Staple Cartridge," issued May 16, 1995; U.S. Pat. No. 5,465,895, entitled "Surgical Stapler Instrument," issued November 14, 1995; U.S. Pat. No. 5,597,107, entitled "Surgical Stapler Instrument," issued January 28, 1997; U.S. Pat. No. 5,632,432, entitled "Surgical Instrument," issued May 27, 1997; U.S. Pat. No. 5,673,840, entitled "Surgical Instrument," issued October 7, 1997; U.S. Pat. No. 5,704,534, entitled "Articulation Assembly for Surgical Instruments," issued January 6, 1998; U.S. Pat. No. 5,814,055, entitled "Surgical Clamping Mechanism," issued September 29, 1998; U.S. Pat. No. 6,978,921, entitled "Surgical Stapling Instrument Incorporating an E-Beam Firing Mechanism," issued December 27, 2005; U.S. Pat. No. 7,000,818, entitled "Surgical Stapling Instrument Having Separate Distinct Closing and Firing Systems," issued February 21, 2006; U.S. Pat. No. 7,143,923, entitled "Surgical Stapling Instrument Having a Firing Lockout for an Unclosed Anvil," issued December 5, 2006; U.S. Pat. No. 7,303,108, entitled "Surgical Stapling Instrument Incorporating a Multi-Stroke Firing Mechanism with a Flexible Rack," issued December 4, 2007; U.S. Pat. No. 7,367,485, entitled "Surgical Stapling Instrument Incorporating a Multistroke Firing Mechanism Having a Rotary Transmission," issued May 6, 2008; U.S. Pat. No. 7,380,695, entitled "Surgical Stapling Instrument Having a Single Lockout Mechanism for Prevention of Firing," issued June 3, 2008; U.S. Pat. No. 7,380,696, entitled "Articulating Surgical Stapling Instrument Incorporating a Two-Piece E-Beam Firing Mechanism," issued June 3, 2008; U.S. Pat. No. 7,404,508, entitled "Surgical Stapling and Cutting Device," issued July 29, 2008; U.S. Pat. No. 7,434,715, entitled "Surgical Stapling Instrument Having Multistroke Firing with Opening Lockout," issued October 14, 2008; and U.S. Pat. No. 7,721,930, entitled "Disposable Cartridge with Adhesive for Use with a Stapling Device," issued May 25, 2010. While the surgical staplers referred to above are described as being used in endoscopic procedures, it should be understood that such surgical staplers may also be used in open procedures and/or other non-endoscopic procedures.

While various kinds of surgical stapling instruments and associated components have been made and used, it is believed that no one prior to the inventor(s) has made or used the invention described in the appended claims.

The invention relates to an apparatus as defined in claim 1. Preferred embodiments are specifed in the dependent claims.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1A depicts a perspective view of an articulating surgical instrument with an end effector in a nonarticulated position;
FIG. 1B depicts a perspective view of the surgical instrument of FIG. 1A with an end effector in an articulated position;
FIG. 2 depicts a perspective view of an opened end effector of the surgical instrument of FIGS. 1A-1B;
FIG. 3A depicts a side cross-sectional view of the end effector of FIG. 2, taken along line 3-3 of FIG. 2, with the firing bar in a proximal position;
FIG. 3B depicts a side cross-sectional view of the end effector of FIG. 2, taken along line 3-3 of FIG. 2, but showing the firing bar in a distal position;
FIG. 4 depicts an end cross-sectional view of the end effector of FIG. 2, taken along line 4-4 of FIG. 2;
FIG. 5 depicts an exploded perspective view of the end effector of FIG. 2;
FIG. 6 depicts a perspective view of the end effector of FIG. 2, positioned at tissue and having been actuated once in the tissue;
FIG. 7 depicts a perspective view of a retainer cap with the end effector of FIG. 2;
FIG. 8 depicts a perspective underside view of the retainer cap of FIG. 7;
FIG. 9 depicts a side view of the retainer cap with the end effector of FIG. 7;
FIG. 10 depicts a top perspective view of an exemplary retainer cap with a buttress material;
FIG. 11A depicts a side view of the retainer cap with the buttress material of FIG. 10 being loaded into an end effector;
FIG. 11B depicts a side view of the retainer cap with the buttress material of FIG. 10, the retainer cap being removed from the end effector, leaving the buttress material loaded in the end effector;
FIG. 12A depicts a cross sectional view of an anvil of the end effector with the buttress material of FIG. 10 where the buttress material is coupled to the anvil through a tab engaging an anvil slot;
FIG. 12B depicts a cross sectional view of an anvil of the end effector with the buttress material of FIG. 10 where the tab and tissue are being severed by a cutter;
FIG. 12C depicts a series cross sectional view of an anvil of the end effector with the buttress material of FIG. 10 where the anvil is being removed from the severed tissue leaving behind the buttress material;
FIG. 13 depicts a front, cross sectional view of an exemplary alternative version of an anvil and an exemplary alternative version of tabs having a T-shape;
FIG. 14 depicts a front, cross-sectional view of another exemplary alternative version of an anvil having notches and another exemplary alternative version of tabs; and
FIG. 15 depicts a perspective view of the buttress material of FIG. 10 having been deployed in tissue during use of a surgical instrument.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

### I. Exemplary Surgical Stapler

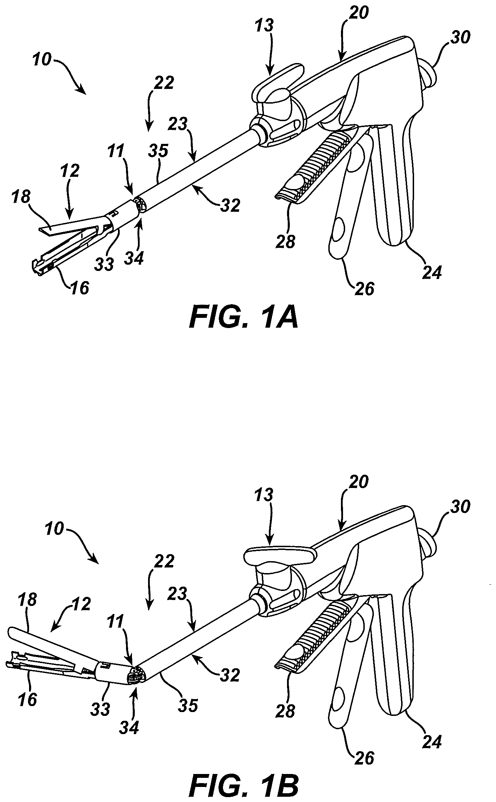

FIGS. 1-6 depict an exemplary surgical stapling and severing instrument (10) that is sized for insertion, in a nonarticulated state as depicted in FIG. 1A, through a trocar cannula passageway to a surgical site in a patient for performing a surgical procedure. Surgical stapling and severing instrument (10) includes handle portion (20) connected to implement portion (22), the latter further comprising shaft (23) distally terminating in an articulation mechanism (11) and a distally attached end effector (12). Once articulation mechanism (11) and end effector (12) are inserted through the cannula passageway of a trocar, articulation mechanism (11) may be remotely articulated, as depicted in FIG. 1B, by articulation control (13). Thereby, end effector (12) may reach behind an organ or approach tissue from a desired angle or for other reasons. It should be understood that terms such as "proximal" and "distal" are used herein with reference to a clinician gripping handle portion (20) of instrument (10). Thus, end effector (12) is distal with respect to the more proximal handle portion (20). It will be further appreciated that for convenience and clarity, spatial terms such as "vertical" and "horizontal" are used herein with respect to the drawings. However, surgical instruments are used in many orientations and positions, and these terms are not intended to be limiting and absolute.

End effector (12) of the present example includes a lower jaw (16) and a pivotable anvil (18). Handle portion (20) includes pistol grip (24) toward which closure trigger (26) is pivotally drawn by the clinician to cause clamping, or closing, of the anvil (18) toward lower jaw (16) of end effector (12). Such closing of anvil (18) is provided through an outmost closure sleeve (32), which longitudinally translates relative to handle portion (20) in response to pivoting of closure trigger (26) relative to pistol grip (24). A distal closure ring (33) of closure sleeve (32) is indirectly supported by frame (34) of implement portion (22). At articulation mechanism (11), a proximal closure tube (35) of closure sleeve (32) communicates with the distal closure ring (33). Frame (34) is flexibly attached to lower jaw (16) via articulation mechanism (11), enabling articulation in a single plane. Frame (34) also longitudinally slidingly supports a firing drive member (not shown) that extends through shaft (23) and communicates a firing motion from firing trigger (28) to firing bar (14). Firing trigger (28) is farther outboard of closure trigger (26) and is pivotally drawn by the clinician to cause the stapling and severing of clamped tissue in end effector (12), as will be described in greater detail below. Thereafter, release button (30) is depressed to release the tissue from end effector (12).

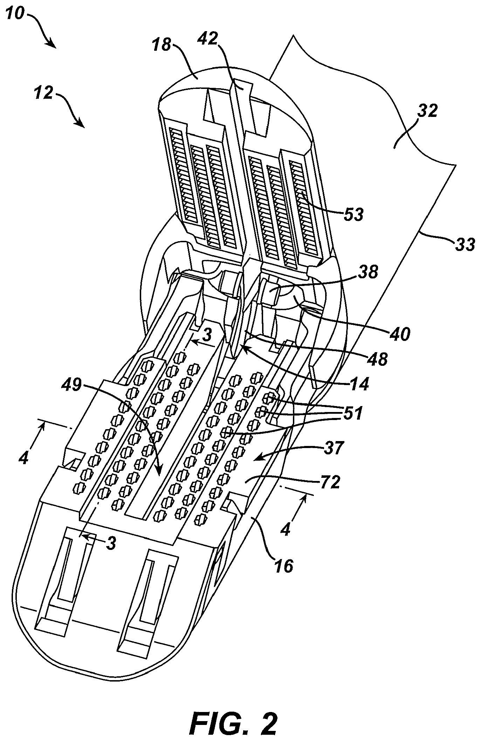

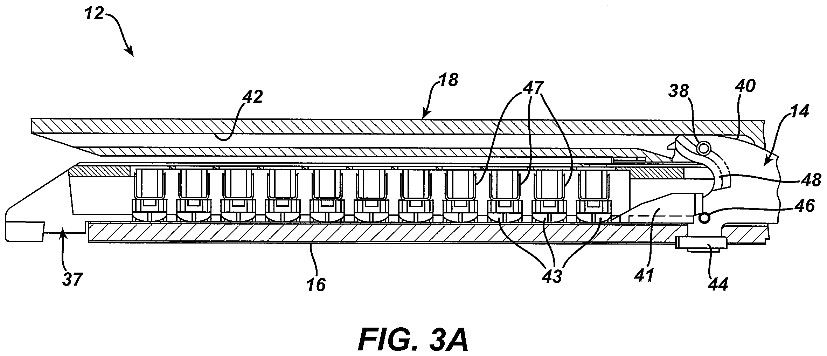

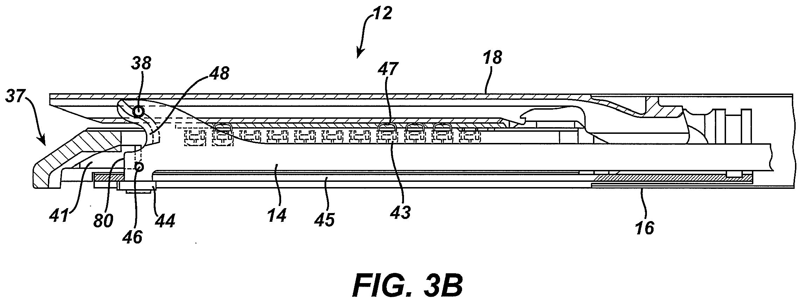

FIGS. 2-5 depict end effector (12) employing an E-beam firing bar (14) to perform a number of functions. As best seen in FIGS. 3A-3B, firing bar (14) includes a transversely oriented upper pin (38), a firing bar cap (44), a transversely oriented middle pin (46), and a distally presented cutting edge (48). Upper pin (38) is positioned and translatable within an anvil pocket (40) of anvil (18). Firing bar cap (44) slidably engages a lower surface of lower jaw (16) by having firing bar (14) extend through channel slot (45) (shown in FIG. 3B) that is formed through lower jaw (16). Middle pin (46) slidingly engages a top surface of lower jaw (16), cooperating with firing bar cap (44). Thereby, firing bar (14) affirmatively spaces end effector (12) during firing, overcoming pinching that may occur between anvil (18) and lower jaw (16) with a minimal amount of clamped tissue and overcoming staple malformation with an excessive amount of clamped tissue.

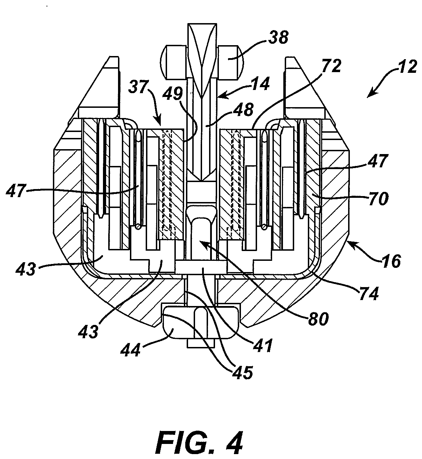

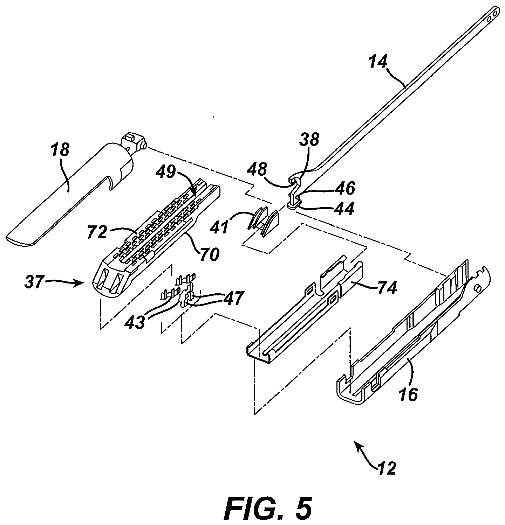

FIG. 2 shows firing bar (14) proximally positioned and anvil (18) pivoted to an open position, allowing an unspent staple cartridge (37) to be removably installed into a channel of lower jaw (16). As best seen in FIGS. 4-5, staple cartridge (37) of this example includes a cartridge body (70), which presents an upper deck (72) and is coupled with a lower cartridge tray (74). As best seen in FIG. 2, a vertical slot (49) is formed through part of staple cartridge (37). As also best seen in FIG. 2, three rows of staple apertures (51) are formed through upper deck (72) on one side of vertical slot (49), with another set of three rows of staple apertures (51) being formed through upper deck (72) on the other side of vertical slot (49). Referring back to FIGS. 3-5, a wedge sled (41) and a plurality of staple drivers (43) are captured between cartridge body (70) and tray (74), with wedge sled (41) being located proximal to staple drivers (43). Wedge sled (41) is movable longitudinally within staple cartridge (37); while staple drivers (43) are movable vertically within staple cartridge (37). Staples (47) are also positioned within cartridge body (70), above corresponding staple drivers (43). In particular, each staple (47) is driven vertically within cartridge body (70) by a staple driver (43) to drive staple (47) out through an associated staple aperture (51). As best seen in FIGS. 3A-3B and 5, wedge sled (41) presents inclined cam surfaces that urge staple drivers (43) upwardly as wedge sled (41) is driven distally through staple cartridge (37).

With end effector (12) closed as depicted in FIG. 3A, firing bar (14) is advanced in engagement with anvil (18) by having upper pin (38) enter a longitudinal anvil slot (42). A pusher block (80) is located at the distal end of firing bar (14), and is configured to engage wedge sled (41) such that wedge sled (41) is pushed distally by pusher block (80) as firing bar (14) is advanced distally through staple cartridge (37). During such firing, cutting edge (48) of firing bar (14) enters vertical slot (49) of staple cartridge (37), severing tissue clamped between staple cartridge (37) and anvil (18). As shown in FIGS. 3A-3B, middle pin (46) and pusher block (80) together actuate staple cartridge (37) by entering into slot (49) within staple cartridge (37), driving wedge sled (41) into upward camming contact with staple drivers (43) that in turn drive staples (47) out through staple apertures (51) and into forming contact with staple forming pockets (53) on the inner surface of anvil (18). FIG. 3B depicts firing bar (14) fully distally translated after completing severing and stapling tissue.

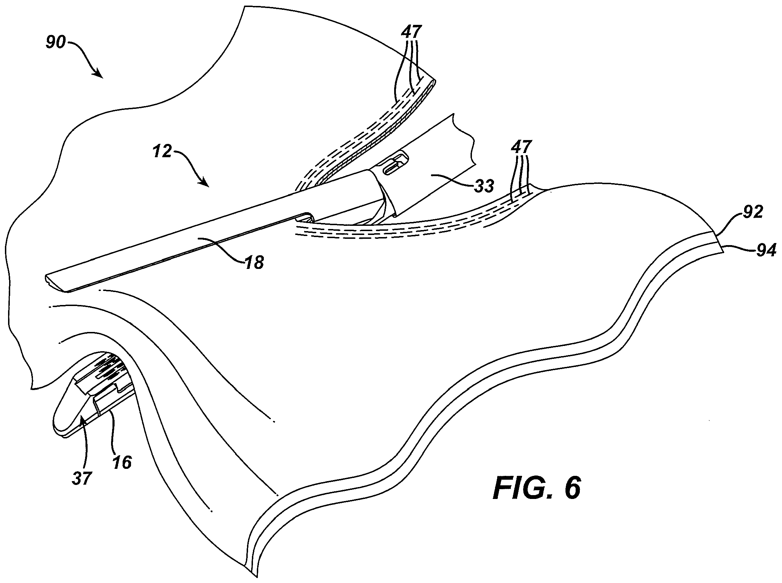

FIG. 6 shows end effector (12) having been actuated through a single stroke through tissue (90). Cutting edge (48) has cut through tissue (90), while staple drivers (43) have driven three alternating rows of staples (47) through the tissue (90) on each side of the cut line produced by cutting edge (48). Staples (47) are all oriented substantially parallel to the cut line in this example, though it should be understood that staples (47) may be positioned at any suitable orientations. In the present example, end effector (12) is withdrawn from the trocar after the first stroke is complete, spent staple cartridge (37) is replaced with a new staple cartridge, and end effector (12) is then again inserted through the trocar to reach the stapling site for further cutting and stapling. This process may be repeated until the desired amount of cuts and staples (47) have been provided. Anvil (18) may need to be closed to facilitate insertion and withdrawal through the trocar; and anvil (18) may need to be opened to facilitate replacement of staple cartridge (37).

It should be understood that cutting edge (48) may sever tissue substantially contemporaneously with staples (47) being driven through tissue during each actuation stroke. In the present example, cutting edge (48) just slightly lags behind driving of staples (47), such that a staple (47) is driven through the tissue just before cutting edge (48) passes through the same region of tissue, though it should be understood that this order may be reversed or that cutting edge (48) may be directly synchronized with adjacent staples. While FIG. 6 shows end effector (12) being actuated in two layers (92, 94) of tissue (90), it should be understood that end effector (12) may be actuated through a single layer of tissue (90) or more than two layers (92, 94) of tissue. It should also be understood that the formation and positioning of staples (47) adjacent to the cut line produced by cutting edge (48) may substantially seal the tissue at the cut line, thereby reducing or preventing bleeding and/or leaking of other bodily fluids at the cut line. Various suitable settings and procedures in which instrument (10) may be used will be apparent to those of ordinary skill in the art in view of the teachings herein.
It should be understood that instrument (10) may be configured and operable in accordance with any of the teachings of U.S. Pat. No. 4,805,823; U.S. Pat. No. 5,415,334; U.S. Pat. No. 5,465,895; U.S. Pat. No. 5,597,107; U.S. Pat. No. 5,632,432; U.S. Pat. No. 5,673,840; U.S. Pat. No. 5,704,534; U.S. Pat. No. 5,814,055; U.S. Pat. No. 6,978,921; U.S. Pat. No. 7,000,818; U.S. Pat. No. 7,143,923; U.S. Pat. No. 7,303,108; U.S. Pat. No. 7,367,485; U.S. Pat. No. 7,380,695; U.S. Pat. No. 7,380,696; U.S. Pat. No. 7,404,508; U.S. Pat. No. 7,434,715; and/or U.S. Pat. No. 7,721,930.

Additional exemplary modifications that may be provided for instrument (10) will be described in greater detail below. Various suitable ways in which the below teachings may be incorporated into instrument (10) will be apparent to those of ordinary skill in the art. Similarly, various suitable ways in which the below teachings may be combined with various teachings of the patents cited herein will be apparent to those of ordinary skill in the art. It should also be understood that the below teachings are not limited to instrument (10) or devices taught in the patents cited herein. The below teachings may be readily applied to various other kinds of instruments, including instruments that would not be classified as surgical staplers. Various other suitable devices and settings in which the below teachings may be applied will be apparent to those of ordinary skill in the art in view of the teachings herein.

### II. Exemplary Cover

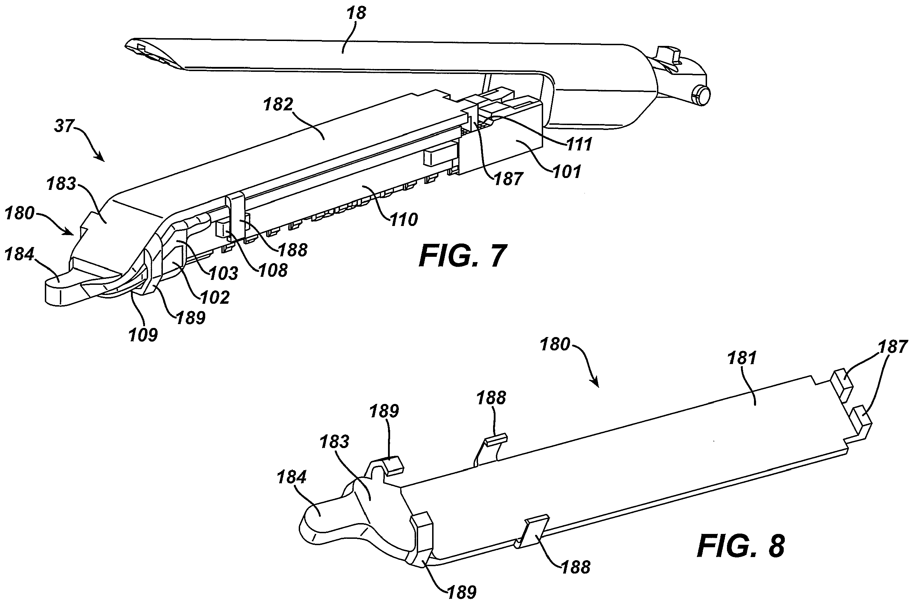

FIGS. 7-9 show an exemplary staple cartridge (37) retainer cap (180), which may be attached to staple cartridge (37). It should be understood that retainer cap (180) may be configured and operable in accordance with any of the teachings of U.S. Patent Application Serial No. 12/894,369, entitled "Implantable Fastener Cartridge Comprising a Support Retainer", filed on September 30, 2010 and/or U.S. Patent Application Serial No. 13/097,924, entitled "Staple Cartridge Comprising a Tissue Thickness Compensator," filed on April 29, 2011. Retainer cap (180) is operable to prevent, or at least inhibit, a clinician's thumb, for example, from contacting the tips of staples (47) positioned within staple cartridge (37) when staple cartridge (37) is inserted into lower jaw (16) of end effector (12). In addition or in the alternative, it may prevent staples (47) from inadvertently falling out of the cartridge. Referring now to FIGS. 7 and 8, retainer cap (180) of the present example includes bottom surface (181) and top surface (182), which can provide a pushing surface for the clinician to apply a downward force thereto, for example. In one merely exemplary use, the clinician may grab handle portion (184) of retainer cap (180), align support portion (110) of staple cartridge (37) with lower jaw (16) of end effector (12), and at least partially insert staple cartridge (37) within lower jaw (16) of end effector (12). Thereafter, the clinician can completely seat staple cartridge (37) in lower jaw (16) of end effector (12) by applying the downward force to top surface (182) of retainer cap (180) which can transmit the downward force directly to support portion (110). In the present example, retainer cap (180) comprises proximal supports (187) which extend downwardly and contact deck surface (111) of the support portion. Retainer cap (180) further comprises distal support portion (183), which abuts nose (103). When a downward force is applied to retainer cap (180), the downward force can be transmitted through proximal supports (187) and/or distal support portion (183). In some exemplary versions, at least some of the supports may not be in contact with the top of support portion (110) before the downward force is applied to retainer cap (180); however, in some versions, retainer cap (180) can be operable to flex, or move, downwardly until retainer cap (180) touches the top of support portion (110). At such point, the downward flexure, or movement, of retainer cap (180) can be impeded, or at least substantially impeded, from flexing further.

As described above, retainer cap (180) can be attached to staple cartridge (37) and can be used to manipulate the position of staple cartridge (37). In some versions, retainer cap (180) can comprise any suitable number of gripping members which can be operable to releasably hold retainer cap (180) to support portion (110) of staple cartridge (37), for example. For instance, in the present example, retainer cap (180) comprises latch arms (188, 189). Latch arms (189) extend around the sides of nose (103) and engage bottom surface (109) (FIG. 7) of nose (103). Similarly, latch arms (188) extend around the sides of lock projections (108) extending from support portion (110) and engage the bottom surfaces of lock projections (108). These latch arms (188) are operable to position retainer cap (180) over the zone or region in which the staples (47) are stored within support portion (110). In any event, once staple cartridge (37) has been suitably positioned, retainer cap (180) can be detached from staple cartridge (37). Of course, any other suitable components or features may be used to provide releasable coupling of retainer cap (180) to staple cartridge (37). In some versions, the clinician may apply an upward lifting force to handle (184) in order to detach the distal end of retainer cap (180) from distal end (102) of staple cartridge (37). In at least one such embodiment, latch arms (188, 189) may flex outwardly as handle (184) is lifted upwardly such that latch arms (188, 189) may flex around lock projections (108) and nose (103), respectively. Thereafter, the proximal end of retainer cap (180) may be lifted away from proximal end (101) of staple cartridge (37) and retainer cap (180) may be moved away from staple cartridge (37). With retainer cap (180) removed and with staple cartridge (37) properly seated in lower jaw (16) of end effector (12), instrument (10) may then be used in a surgical procedure.

### III. Exemplary Buttress Material

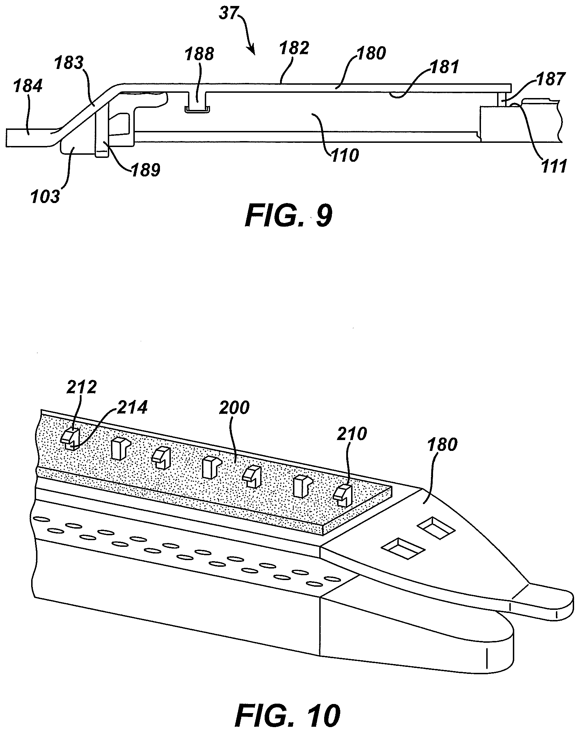

According to the invention, retainer cap (180) is used in conjunction with a buttress material (200) as shown, for example, in FIGS. 10-12C. It may be desirable to utilize buttress material (200) during the severing and/or stapling of tissue to aid in supporting and healing of the severed area. In the present example, buttress material (200) comprises a coagulant operable to buttress a surgical site when the surgical site is severed and stapled. For example, buttress material (200) may comprise fibrin and thrombin, but any suitable coagulant may be used as would be apparent to one of ordinary skill in the art in view of the teachings herein. Furthermore, buttress material (200) may comprise a hemostatic agent, any suitable glue material, acellular tissue (e.g., allogeneic or xenogeneic), collagen, gelatin, oxidized regenerated cellulose (ORC), degradable woven and non-woven synthetic materials such as polylactate, polyglycol, polycuproic acid, or any other suitable material as would be apparent to one of ordinary skill in the art in view of the teachings herein. In some exemplary versions, buttress material (200) may comprise a fibrin pad. Additionally, buttress material (200) may comprise any combination of a variety of suitable materials.

As shown in the exemplary version, buttress material (200) may be placed on the top surface of retainer cap (180) as well as the bottom surface of retainer cap (180) to extend along substantially the length of retainer cap (180). In some exemplary versions, buttress material (200) placed on the top surface of retainer cap (180) has a different material composition from buttress material (200) placed on the bottom surface of retainer cap (180). For example, a portion of buttress material (200) may comprise fibrin wherein the other portion of buttress material (200) could comprise thrombin. However, various suitable materials for buttress material (200) may be used as would be apparent to one of ordinary skill in the art in view of the teachings herein. In the illustrated version, buttress material (200) is generally spread evenly across the top surface and the bottom surface of retainer cap (180), but it will be appreciated that buttress material (200) may be distributed in discrete portions of the top surface and the bottom surface of retainer cap (180). For example, a greater amount of buttress material (200) may be placed on the top surface of retainer cap (180) or a greater amount of buttress material (200) may be placed on the bottom surface of retainer cap (180). Furthermore, buttress material (200) may be placed on only the top surface of retainer cap (180) or the bottom surface of retainer cap (180). Any suitable distribution of buttress material (200) along retainer cap (180) may be used as would be apparent to one of ordinary skill in the art in view of the teachings herein. Buttress material (200) comprises a semi-rigid and/or rigid structure such that when separated from retainer cap (180) as will be described below, buttress material (200) is able to generally maintain its flat, rectangular configuration.

As shown in FIG. 10, a plurality of resilient tabs (210) is positioned along the top surface of buttress material (200). Tabs (210) are spaced apart and aligned generally longitudinally along the center of buttress material (200). Tabs (210) are further arranged to be facing alternating lateral directions along the length of buttress material (200). It will be appreciated that the bottom portion of buttress material (200) may also comprise tabs (210) extending similarly in an opposite direction from tabs (210) positioned on top of buttress material (200).

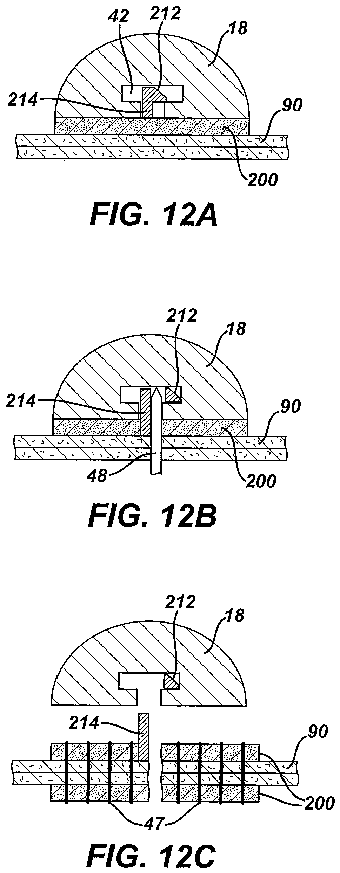

Tabs (210) each comprise a head portion (212) and body portion (214). Tabs (210) are generally aligned to snap into longitudinal anvil slot (42), shown in FIGS. 12A-C, such that buttress (200) becomes mechanically coupled to anvil (18) when retainer cap (180) is pressed against anvil (18). Head portion (212) of tabs (210) has a wedge-like shape such that head portion (212) can be easily urged into longitudinal anvil slot (42) without being removable from longitudinal anvil slot (42) once snapped into place. Tabs (210) are coupled to buttress material (200) such that buttress material (200) may be coupled to anvil (18) by way of tabs (210) coupling with longitudinal anvil slot (42). While the illustrated version depicts tabs (210) as having a wedge-like shape, any suitable shape may be used including a T-shape or a bulb-like shape. Various other suitable shapes for tabs (210) and/or head portion (212) will be apparent to one of ordinary skill in the art in view of the teachings herein.

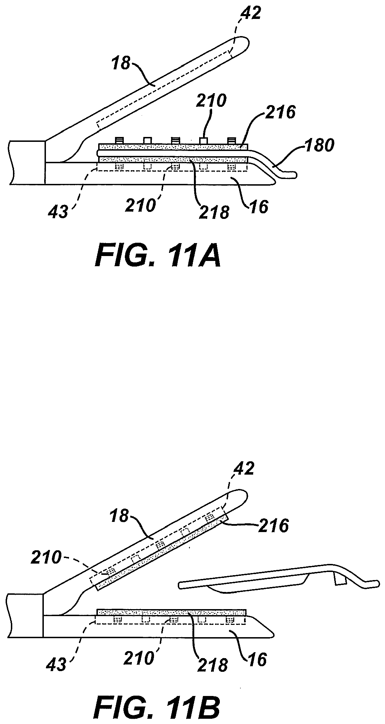

FIG. 11A shows buttress material (200) and retainer cap (180) being loaded into lower jaw (16). Anvil (18) is not yet coupled to buttress material (200) and anvil (18) remains open. Thus, a user may be able to view whether buttress material (200) and retainer cap (180) is properly lined up within lower jaw (16) prior to closing anvil (18) to engage buttress material (200).

As can be seen in FIGS 11B, anvil (18) and lower jaw (16) have closed upon retainer cap (180), which is covered with buttress material (200), thereby causing tabs (210) to snap into longitudinal anvil slot (42). A portion of buttress (200) may also be coupled to lower jaw (16) by attaching to a staple cartridge (not shown) positioned within lower jaw (16). Furthermore, lower jaw (16) comprises a longitudinal lower jaw slot (43) similarly shaped to longitudinal anvil slot (42) for engaging tabs (210) of buttress (200). Thereafter, anvil (18) and lower jaw (16) may be opened, leaving a top portion of buttress material (216) coupled to anvil (18) and a bottom portion of buttress material (218) coupled to lower jaw (16). It will be appreciated that retainer cap (180) may be held between anvil (18) and lower jaw (16) until instrument (10) is ready for use. It will also be appreciated that other suitable configurations may be used. For example, buttress material (200) may be coupled to only anvil (18). In other merely exemplary versions, buttress material (200) may be coupled to only lower jaw (16). In yet other exemplary versions, buttress material (200) may be directly coupled to lower jaw (16) and/or anvil (18) without the use of retainer cap (180). In some versions, buttress material (200) and retainer cap (180) may be pre-loaded into anvil (18) and lower jaw (16) such that the user need only remove retainer cap (180) prior to use.

FIGS. 12A-C show a series of cross-sectional views of anvil (18) coupled with buttress material (200) with tabs (210) snapped into longitudinal anvil slot (42) with tissue (90) pressed against buttress (200). FIG. 12A shows buttress (200) loaded into anvil (18) with tabs (210) pressed into anvil slot (42). Head portion (212) is operable to prevent buttress (200) from inadvertently decoupling from anvil (18).

FIG. 12B shows cutting edge (48), which is shown in FIGS. 1-6, as it travels along the length of longitudinal anvil slot (42). Cutting edge (48) slices through head portion (212) of tabs (210), thereby decoupling tabs (210) from longitudinal anvil slot (42). As can be seen in FIG. 12B, body portion (214) of each of tabs (210) is slightly off center such that only head portion (212) is severed as cutting edge (48) advances through longitudinal anvil slot (42). As cutting edge (48) advances, cutting edge (48) severs tissue (90) such that tissue (90) and tabs (210) are severed substantially contemporaneously. Additionally, as cutting edge (48) advances, an upper pin, which is shown for example in FIG. 3A, travels with cutting edge (48) and urges head portion (212) out of anvil slot (42). Tabs (210) comprise a biodegradable material such that portions of tabs (210) released into tissue (90) ultimately dissolve or otherwise remain biologically inert without exposing tissue (90) to any danger.

As can be seen in FIG. 12C, as cutting edge (48) slices through tissue (90), buttress material (200) is stapled to tissue (90) with staples (47), and furthermore supports and/or heals the surrounding tissue (90) and/or reinforces the line of staples (47). In the exemplary version, body portion (214) of tabs (210) may remain coupled to buttress material (200) and/or may simply dissolve as the cut tissue heals. Furthermore, as tissue (90) material heals, buttress material (200) may dissolve. In some alternative versions, buttress material (200) may be removed once tissue (90) has sufficiently healed.

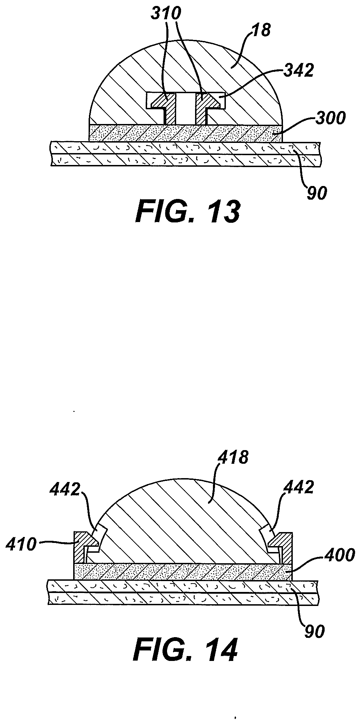

FIG. 13 shows another merely exemplary version of tabs (310), not forming part of the invention, having a split T-shape and extending continuously along the length of buttress material (300). Rather than snapping into the complementary shaped longitudinal anvil slot (342), tabs (310) in communication with buttress material (300) may be slid longitudinally into complementary shaped longitudinal anvil slot (342). In other exemplary versions, tabs (310) may have a split mushroom shape. It will be appreciated that the split mushroom shape may be operable to provide easier inserting of tabs (310) into anvil slot (342) by having a slightly rounded portion. In either the split T-shape or the split mushroom-shape configuration, it will be appreciated that as cutting edge (48) advances to sever tissue (90), as described for example, in FIGS. 1-6, cutting edge (48) may deflect and/or deform tabs (310) such that tabs (310) may be removed easily from anvil slot (342) as a result of tabs (310) being deformed. In yet other exemplary versions, as cutting edge (48) advances to sever tissue (90), cutting edge (48) may shear off a portion and/or the entirety of tabs (310) sufficient to cause tabs (310) to disengage anvil slot (342).

In yet another exemplary version shown in FIG. 14, not forming part of the invention, buttress material (400) may be in communication with clips (410) operable to engage a series of corresponding exterior notches (442) on anvil (418) to couple buttress material (400) to anvil (418). Once tissue (90) has been severed and stapled, the user may disengage clips (410) to decouple buttress material (400) from anvil (418). Other exemplary configurations for coupling buttress material (400) to anvil (418) will be apparent to one of ordinary skill in the art in view of the teachings herein.

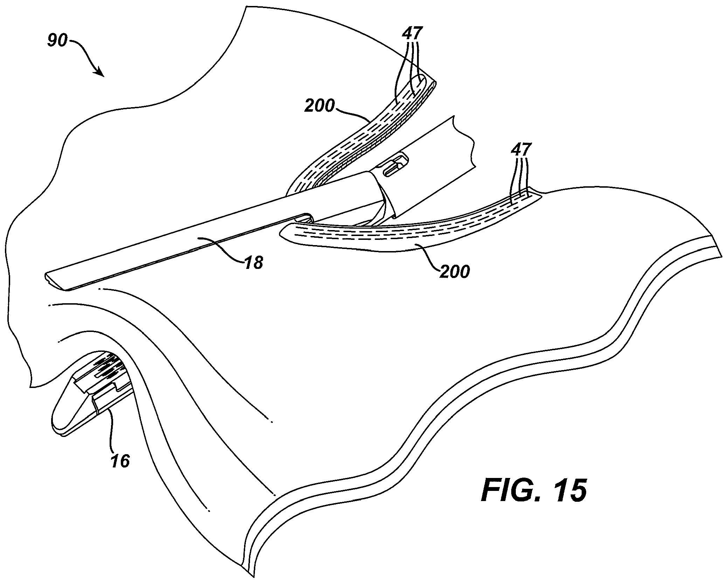

FIG. 15 shows surgical severing and stapling instrument (10) after having been used to sever and staple tissue (90). As can be seen in the present example, buttress (200) has been stapled to tissue (90) and thereafter may aid in recovery of tissue (90) by stabilizing staples (47) to tissue (90).

It should be understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices described above may have application in conventional medical treatments and procedures conducted by a medical professional, as well as application in robotic-assisted medical treatments and procedures.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various versions in the present disclosure, further adaptations of the methods and systems described herein may be accomplished by appropriate modifications by one of ordinary skill in the art without departing from the scope of the appended claims. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus, comprising:
(a) a surgical instrument (10) comprising an anvil (18), wherein the anvil comprises a longitudinal anvil slot (42), wherein the surgical instrument is configured to sever and staple a portion of tissue, and comprises a distally advancing cutter (14), wherein the surgical instrument defines a longitudinal center, wherein the cutter advances substantially along the longitudinal center of the surgical instrument, and wherein the surgical instrument comprises a proximal end and a distal end;
(b) a retainer cap (180) attached to the distal end of the surgical instrument, wherein the retainer cap is operable to cover at least a portion of the distal end of the surgical instrument; and
(c) a buttress material (200) placed on the retainer cap, wherein the buttress material is configured to couple with a portion of the distal end of the surgical instrument, wherein the retainer cap is configured to be removed from the distal end of the surgical instrument without decoupling the buttress material from the portion of the distal end of the surgical instrument, wherein the buttress material is further configured to decouple from the portion of the distal end of the surgical instrument,
**characterised in that** the buttress material is configured to couple with the longitudinal anvil slot, and the buttress material further comprises a plurality of tabs (210) configured to snap into the longitudinal anvil slot, wherein the plurality of tabs are arranged to be slightly offset from the longitudinal center of the surgical instrument, and wherein each of the plurality of tabs comprises a body portion (214) and a head portion (212), wherein the cutter is configured to sever the head portion as the cutter distally advances.

2. The apparatus of claim 1, wherein the plurality of tabs comprise dissolvable tabs.

3. The apparatus of claim 1, wherein the plurality of tabs are arranged to face alternating lateral directions along the length of the buttress material.

4. The apparatus of claim 1, wherein the buttress material comprises a hemostatic agent.

5. The apparatus of claim 1, wherein the buttress material comprises a fibrin pad.

6. The apparatus of claim 1, wherein the distal end of the surgical instrument comprises the anvil and a lower jaw, wherein the anvil and the lower jaw are configured to clamp the buttress material around tissue to be cut and stapled such that the buttress material sandwiches the tissue to be cut and stapled.

7. The apparatus of claim 1, wherein at least a portion of the buttress material comprises a dissolvable material.

8. The apparatus of claim 1, wherein the retainer cap comprises a top surface and a bottom surface, wherein the buttress material covers the top surface of the retainer cap, wherein the buttress material further covers the bottom surface of the retainer cap.

## Patentansprüche

1. Vorrichtung, umfassend:
(a) ein chirurgisches Instrument (10), das einen Amboss (18) umfasst, wobei der Amboss einen länglichen Ambossschlitz (42) umfasst, wobei das chirurgische Instrument dazu ausgestaltet ist, einen Gewebeabschnitt abzutrennen und zu klammern, und eine sich distal vorwärtsbewegende Schneidvorrichtung (14) umfasst, wobei das chirurgische Instrument eine Längsmitte definiert, wobei sich die Schneidvorrichtung im Wesentlichen entlang der Längsmitte des chirurgischen Instruments vorwärtsbewegt und wobei das chirurgische Instrument ein proximales Ende und ein distales Ende umfasst,
(b) eine Haltekappe (180), die an dem distalen Ende des chirurgischen Instruments angebracht ist, wobei die Haltekappe dahingehend betätigbar ist, mindestens einen Abschnitt des distalen Endes des chirurgischen Instruments abzudecken, und
(c) ein Versteifungsmaterial (200), das auf der Haltekappe platziert ist, wobei das Versteifungsmaterial dazu ausgestaltet ist, mit einem Abschnitt des distalen Endes des chirurgischen Instruments zu koppeln, wobei die Haltekappe dazu ausgestaltet ist, ohne Abkoppeln des Versteifungsmaterials von dem Abschnitt des distalen Endes des chirurgischen Instruments von dem distalen Ende des chirurgischen Instruments entfernt zu werden, wobei das Versteifungsmaterial ferner dazu ausgestaltet ist, von dem Abschnitt des distalen Endes des chirurgischen Instruments abgekoppelt zu werden,
**dadurch gekennzeichnet, dass** das Versteifungsmaterial dazu ausgestaltet ist, mit dem länglichen Ambossschlitz zu koppeln, und das Versteifungsmaterial ferner eine Vielzahl von Laschen (210) umfasst, die dazu ausgestaltet sind, in den länglichen Ambossschlitz einzuschnappen, wobei die Vielzahl von Laschen so angeordnet sind, dass sie von der Längsmitte des chirurgischen Instruments leicht versetzt sind, und wobei jede der Vielzahl von Laschen einen Körperabschnitt (214) und einen Kopfabschnitt (212) umfasst, wobei die Schneidvorrichtung dazu ausgestaltet ist, den Kopfabschnitt abzutrennen, während sich die Schneidvorrichtung distal vorwärtsbewegt.

2. Vorrichtung nach Anspruch 1, wobei die Vielzahl von Laschen lösbare Laschen umfassen.

3. Vorrichtung nach Anspruch 1, wobei die Vielzahl von Laschen so angeordnet sind, dass sie in abwechselnde seitliche Richtungen entlang der Länge des Versteifungsmaterials weisen.

4. Vorrichtung nach Anspruch 1, wobei das Versteifungsmaterial ein Hämostatikum umfasst.

5. Vorrichtung nach Anspruch 1, wobei das Versteifungsmaterial eine Fibrin-Auflage umfasst.

6. Vorrichtung nach Anspruch 1, wobei das distale Ende des chirurgischen Instruments den Amboss und eine untere Backe umfasst, wobei der Amboss und die untere Backe dazu ausgestaltet sind, das Versteifungsmaterial um zu schneidendes und zu klammerndes Gewebe herum zu klemmen, so dass das Versteifungsmaterial das zu schneidende und zu klammernde Gewebe einklemmt.

7. Vorrichtung nach Anspruch 1, wobei mindestens ein Abschnitt des Versteifungsmaterials ein lösbares Material umfasst.

8. Vorrichtung nach Anspruch 1, wobei die Haltekappe eine obere Fläche und eine untere Fläche umfasst, wobei das Versteifungsmaterial die obere Fläche der Haltekappe abdeckt, wobei das Versteifungsmaterial ferner die untere Fläche der Haltekappe abdeckt.

## Revendications

1. Appareil, comprenant :
(a) un instrument chirurgical (10) comprenant une enclume (18), dans lequel l'enclume comprend une fente longitudinale (42) d'enclume, l'instrument chirurgical étant configuré pour découper et agrafer une partie de tissu, et comprenant un instrument tranchant avançant distalement (14), l'instrument chirurgical définissant un centre longitudinal, dans lequel l'instrument tranchant avance sensiblement le long du centre longitudinal de l'instrument chirurgical, et l'instrument chirurgical comprenant une extrémité proximale et une extrémité distale ;
(b) un capuchon de retenue (180) fixé à l'extrémité distale de l'instrument chirurgical, le capuchon de retenue pouvant fonctionner pour couvrir au moins une partie de l'extrémité distale de l'instrument chirurgical ; et
(c) un matériau de renfort (200) placé sur le capuchon de retenue, le matériau de renfort étant conçu pour s'accoupler avec une partie de l'extrémité distale de l'instrument chirurgical, dans lequel le capuchon de retenue est conçu pour être retiré de l'extrémité distale de l'instrument chirurgical sans désolidariser le matériau de renfort de la partie de l'extrémité distale de l'instrument chirurgical, le matériau de renfort étant en outre conçu pour se désolidariser de la partie de l'extrémité distale de l'instrument chirurgical,
**caractérisé en ce que** le matériau de renfort est conçu pour s'accoupler avec la fente longitudinale d'enclume, et le matériau de renfort comprend en outre une pluralité de languettes (210) conçues pour s'encliqueter dans la fente longitudinale d'enclume, dans lequel la pluralité de languettes est conçue pour être légèrement décalée du centre longitudinal de l'instrument chirurgical, et dans lequel chaque languette de la pluralité de languettes comprend une partie corps (214) et une partie tête (212), dans lequel l'instrument tranchant est conçu pour découper la partie tête à mesure que l'instrument tranchant avance distalement.

2. Appareil selon la revendication 1, dans lequel la pluralité de languettes comprend des languettes solubles.

3. Appareil selon la revendication 1, dans lequel la pluralité de languettes est conçue pour faire face à des directions latérales alternées sur la longueur du matériau de renfort.

4. Appareil selon la revendication 1, dans lequel le matériau de renfort comprend un agent hémostatique.

5. Appareil selon la revendication 1, dans lequel le matériau de renfort comprend une pastille de fibrine.

6. Appareil selon la revendication 1, dans lequel l'extrémité distale de l'instrument chirurgical comprend l'enclume et une mâchoire inférieure, dans lequel l'enclume et la mâchoire inférieure sont conçues pour serrer le matériau de renfort autour du tissu devant être coupé et agrafé de sorte que le matériau de renfort prenne en sandwich le tissu devant être coupé et agrafé.

7. Appareil selon la revendication 1, dans lequel au moins une partie du matériau de renfort comprend un matériau soluble.

8. Appareil selon la revendication 1, dans lequel le capuchon de retenue comprend une surface supérieure et une surface inférieure, dans lequel le matériau de renfort couvre la surface supérieure du capuchon de retenue, dans lequel le matériau de renfort couvre en outre la surface inférieure du capuchon de retenue.
